# EUROPEAN PATENT SPECIFICATION

(11) **EP 0 733 634 B1**
(45) Date of publication and mention of the grant of the patent: **22.11.2000**
(21) Application number: 96301999.7
(22) Date of filing: 22.03.1996
(51) Int. Cl.: C07D 495/04, C07B 63/00, A61K 31/55

(54) **Process for preparing a thieno(2,3-B)(1,5) benzodiazepine derivative**
Verfahren zur Herstellung eines Thieno(2,3-B)(1,5)benzodiazepinderivats
Procédé de préparation d'un dérivé de thieno(2,3-B)(1,5)benzodiazepine

(30) Priority: 24.03.1995 US 409566; 24.03.1995 US 410474
(43) Date of publication of application: 25.09.1996
(73) Proprietor: ELI LILLY AND COMPANY, Indianapolis, Indiana 46285 (US); ELI LILLY AND COMPANY LIMITED, Basingstoke, Hants RG21 6XA (GB)
(72) Inventor: Bunnell, Charles Arthur, Lafayette, Indiana 47905 (US); Hendriksen, Barry Arnold, Guildford, Surrey GU2 6QE (GB); Hotten, Terrence Michael, Cove, Farnborough, Hampshire GU14 8PL (GB); Larsen, Samuel Dean, West Lafayette, Indiana 47906 (US); Tupper, David Edward, Reading, Berkshire RG6 3AT (GB)
(74) Representative: Hudson, Christopher Mark

(56) References cited:
- EP-A- 0 454 436
- EP-A- 0 582 368
- US-A- 5 457 101

## Description

This invention relates to a novel process for preparing 2-methyl-4-(4-methyl-1-piperazinyl)-10H-thieno[2,3-b][1,5]benzodiazepine (hereinafter referred to by its generic name "olanzapine"), and to novel alcohol solvates of value in that process.

Olanzapine has shown great promise in the treatment of psychotic patients and is currently being evaluated for that purpose. Unfortunately, the processes of recovery of olanzapine known heretofore have not been entirely satisfactory.

Thus, in the literature methods for preparing olanzapine, such as contained in U.S. Patent No. 5,229,382, technical grade olanzapine was precipitated by the addition of an excess of water to the reaction mixture. Applicants have now discovered that precipitation of technical grade olanzapine using an alcohol solvate selected from the group consisting of methanol, ethanol, and 1-propanol results in a significantly purer technical grade olanzapine requiring only one recrystallization.

Thus, Applicants have now prepared and characterized three stable olanzapine solvates selected from the group consisting of ethanol, methanol, and 1-propanol (herein referred to as "lower alcohol solvates") each of which are particularly useful for preparing the technical grade olanzapine.

Surprisingly, the use of the lower alcohol solvates provides greater yields of the desired technical grade olanzapine, eleminates tedious separation steps, and provides a technical grade material that is easy to handle for production purposes.

The present invention provides a compound selected from the group consisting of a methanol, ethanol, and 1-propanol crystalline solvates of 2-methyl-4-(4-methyl-1-piperazinyl)-10H-thieno[2,3-b][1,5]benzodiazepine (olanzapine).

Further, this invention provides a process for preparing technical grade olanzapine comprising
crystallizing olanzapine from a reaction mixture using a lower alcohol selected from the group consisting of methanol, ethanol, and 1-propanol to provide the corresponding alcohol solvate of olanzapine; and drying the resulting solvate.

As used herein, the term "technical grade 2-methyl-4-(4-methyl-1-piperazinyl)-10H-thieno[2,3-b][1,5]benzodiazepine " shall refer to 2-methyl-4-(4-methyl-1-piperazinyl)-10H-thieno[2,3-b][1,5]benzodiazepine product which contains less than about 5% undesired related substances and preferably less than about 1% undesired related substances.

As used herein, the term "solvate" shall refer to a true solvate of olanzapine, wherein the solvent molecule is held within the crystalline latice.

Form II olanzapine polymorph having a typical x-ray powder diffraction pattern as follows, using a Siemens D5000 x-ray powder diffractometer wherein **d** represents the interplanar spacing:

| **d** |
|---|
| 10.2689 |
| 8.577 |
| 7.4721 |
| 7.125 |
| 6.1459 |
| 6.071 |
| 5.4849 |
| 5.2181 |
| 5.1251 |
| 4.9874 |
| 4.7665 |
| 4.7158 |
| 4.4787 |
| 4.3307 |
| 4.2294 |
| 4.141 |
| 3.9873 |
| 3.7206 |
| 3.5645 |
| 3.5366 |
| 3.3828 |
| 3.2516 |
| 3.134 |
| 3.0848 |
| 3.0638 |
| 3.0111 |
| 2.8739 |
| 2.8102 |
| 2.7217 |
| 2.6432 |
| 2.6007 |

A preferred embodiment of the invention is the crystalline mono(methanol) solvate of 2-methyl-4-(4-methyl-1-piperazinyl)-10H-thieno[2,3-b][1,5]benzodiazepine having a typical x-ray powder diffraction pattern of Table 1:

**TABLE 1**

| Mono(methanol) Solvate | |
|---|---|
| **d** | **I/I**_{**1**} |
| 10.2932 | 100.00 |
| 9.4747 | 3.09 |
| 7.1794 | 0.97 |
| 7.0601 | 2.31 |
| 6.2612 | 1.89 |
| 5.7558 | 1.27 |
| 5.4197 | 5.36 |
| 5.2317 | 1.32 |
| 5.1348 | 0.46 |
| 5.1285 | 1.04 |
| 5.0506 | 1.57 |
| 5.0331 | 1.43 |
| 4.7672 | 2.22 |
| 4.7137 | 7.46 |
| 4.4533 | 6.03 |
| 4.3315 | 3.28 |
| 4.2656 | 2.66 |
| 4.044 | 3.37 |
| 3.9821 | 0.95 |
| 3.9696 | 1.20 |
| 3.9532 | 0.88 |
| 3.9125 | 1.09 |
| 3.8562 | 7.85 |
| 3.7983 | 4.23 |
| 3.7378 | 1.34 |
| 3.7059 | 3.03 |
| 3.6384 | 1.06 |
| 3.6028 | 5.36 |
| 3.5216 | 2.75 |
| 3.4454 | 0.62 |
| 3.4321 | 0.53 |
| 3.4193 | 0.60 |
| 3.248 | 0.90 |
| 3.2416 | 1.18 |
| 3.1347 | 0.88 |
| 3.127 | 0.74 |
| 3.0121 | 0.65 |
| 2.9979 | 0.83 |
| 2.9767 | 0.92 |
| 2.9303 | 0.53 |
| 2.9172 | 0.58 |
| 2.8048 | 0.55 |
| 2.7483 | 0.79 |
| 2.7412 | 0.83 |

The x-ray powder diffraction pattern in Table I and for Form II was obtained with a copper K_{α} of wavelength λ = 1.54184Å. The interplanar spacings are in the column marked "d" are in Angstroms. The typical relative intensities are in the column marked "I/I₁".

Another preferred embodiment of the present invention is the crystalline mono(ethanol)solvate of 2-methyl-4-(4-methyl-1-piperazinyl)-10H-thieno[2,3-b][1,5]benzodiazepine exhibiting a typical x-ray powder diffraction pattern of Table 2:

**TABLE 2**

| Mono(ethanol)solvate | |
|---|---|
| **d** | **I/I**_{**1**} |
| 10.2672 | 100.00 |
| 9.6467 | 1.48 |
| 7.2319 | 2.92 |
| 6.5065 | 1.61 |
| 5.9172 | 0.69 |
| 5.6475 | 1.02 |
| 5.5197 | 2.48 |
| 5.1492 | 4.20 |
| 4.7926 | 2.82 |
| 4.6477 | 0.54 |
| 4.4450 | 4.95 |
| 4.3473 | 2.04 |
| 4.2726 | 0.55 |
| 4.1290 | 2.84 |
| 3.9908 | 2.90 |
| 3.8338 | 1.83 |
| 3.6932 | 1.61 |
| 3.6189 | 1.52 |
| 3.5070 | 0.63 |
| 3.4389 | 8.58 |
| 3.3083 | 0.80 |
| 3.2618 | 0.55 |
| 3.1950 | 0.91 |
| 3.1050 | 0.65 |
| 3.0761 | 0.54 |
| 3.0477 | 0.47 |
| 2.9835 | 0.60 |
| 2.9292 | 0.54 |
| 2.8703 | 0.58 |
| 2.8215 | 0.59 |
| 2.7852 | 2.59 |
| 2.6908 | 0.57 |
| 2.6287 | 0.75 |
| 2.5791 | 5.54 |

Another preferred embodiment of the invention is the crystalline mono(1-propanol)solvate of 2-methyl-4-(4-methyl-1-piperazinyl)-lOH-thieno[2,3-b][1,5]benzodiazepine exhibiting a typical x-ray diffraction pattern of Table 3:

**TABLE 3**

| Mono(1-propanol)solvate | |
|---|---|
| **d** | **I/I**_{**1**} |
| 10.2736 | 100.00 |
| 9.6086 | 1.13 |
| 7.9084 | 0.72 |
| 7.3331 | 2.41 |
| 6.6530 | 5.33 |
| 6.1030 | 0.79 |
| 5.6758 | 6.47 |
| 5.2990 | 1.70 |
| 5.1333 | 1.98 |
| 4.9632 | 14.61 |
| 4.8014 | 3.14 |
| 4.6691 | 2.22 |
| 4.5756 | 1.62 |
| 4.4451 | 9.51 |
| 4.1821 | 1.03 |
| 4.0883 | 3.58 |
| 3.9890 | 1.26 |
| 3.9021 | 1.80 |
| 3.8234 | 3.38 |
| 3.7258 | 1.22 |
| 3.6785 | 1.27 |
| 3.6033 | 1.22 |
| 3.5265 | 0.94 |
| 3.4234 | 1.85 |
| 3.3653 | 0.48 |
| 3.3121 | 1.38 |
| 3.2125 | 0.67 |
| 3.1795 | 0.61 |
| 3.1230 | 0.47 |
| 3.0435 | 1.06 |
| 2.9461 | 1.74 |
| 2.8951 | 0.81 |
| 2.8495 | 1.68 |
| 2.7744 | 0.66 |
| 2.7445 | 0.62 |
| 2.7140 | 0.47 |
| 2.6609 | 0.48 |
| 2.6110 | 0.68 |
| 2.5683 | 2.36 |

The x-ray powder diffraction data in Tables 2 and 3 was collected employing an Enraf-Nonius CAD4 kappa axis diffractometer. The diffraction pattern was obtained with a copper k of wavelength = 1.542A. The interplanar spacings in the column marked "d" are expressed in Angstroms. The typical relative intensities are in the column marked "I/I₁".

The compounds and processes of the present invention are useful for preparing olanzapine. Certain compounds and conditions within the scope of this invention are preferred. The following conditions, invention embodiments, and compound characteristics listed in tabular form may be independently combined to produce a variety of preferred compounds and process conditions. The following list of embodiments of this invention is not intended to limit the scope of this invention in any way.

Some preferred characteristics of this invention include the following:
A) A mono (ethanol) solvate of 2-methyl-4-(4-methyl-1-piperazinyl)-10H-thieno[2,3-b][1,5]benzodiazepine
B) Substantially pure refers to ≤ 5% undesired polymorph;
C) Substantially pure refers to < 2% undesired polymorph;
D) A mono (methanol) solvate of olanzapine;
E) A mono (1-propanol) solvate of olanzapine;
F) A substantially pure solvate of olanzapine selected from the group consisting of mono (methanol), mono (ethanol), and mono (1-propanol).

The formation of solvates is known to be an individualistic effect. The ability of a given compound to form a solvate is not predictable, to Applicant's knowledge. Further, the beneficial utility of such solvates is particularly surprising. The present invention provides three crystalline solvates of 2-methyl-4-(4-methyl-1-piperazinyl)-10H-thieno[2,3-b][1,5]benzodiazepine which have been verified using x-ray crystal techniques. The solvates of this invention may be prepared by suspending 2-methyl-4-(4-methyl-1-piperazinyl)-10H-thieno[2,3-b][1,5]benzodiazepine in warm solvent corresponding to the desired solvate. Most preferably the solvent temperature is from about 0°C to 100°C, although other reaction temperatures can be effective with alteration of the reaction conditions using known techniques. A preferred temperature is from about 25 °C to about 50°C. The mixture is most desirably stirred for about 30 minutes or more. The reaction time will vary with the temperature of the reaction, pressure, and with the completion of reaction desired.

The present invention provides a new method for preparing material which applicants have identified herein as "technical grade olanzapine". The process provides greater yields of the desired material, eleminates tedious separation steps, and provides a technical grade olanzapine that is easy to handle for production purposes.

The most preferred alcohols for forming an olanzapine solvate are methanol and ethanol. A particularly preferred alcohol is methanol. The improved features include fewer recrystallizations, improved throughput, and enhanced environmental safety.

The solvate may be isolated by cooling the mixture to ambient temperature or through the use of an antisolvent. However, the most preferred isolation method is the lower alcohol process described *supra.*

As used herein the term "drying" the solvates shall mean that the solvates are dried or azeotroped under ambient conditions or at a temperature less than about 50°C. The drying may be completed *in vacuo* if the temperature and pressure are carefully monitored using techniques known to the artisan.

The term "crystallized" as used herein refers to any conventional process including, for example, seeding, chilling, scratching the glass of the reaction vessel, and other such common techniques familiar to the skilled artisan.

Compound characterization methods include, for example, x-ray powder pattern analysis, thermogravimetric analysis (TGA), differential scanning calorimetery (DSC), titrametric analysis for water, and H¹-NMR analysis for solvent content.

The following examples are provided for purposes of illustration. As used in the following examples, the term "technical grade 2-methyl-4-(4-methyl-1-piperazinyl)-10H-thieno[2,3-b][1,5]benzodiazepine " shall refer to 2-methyl-4-(4-methyl-1-piperazinyl)-10H-thieno[2,3-b][1,5]benzodiazepine dried solvate product.

### Example 1

### Technical Grade olanzapine

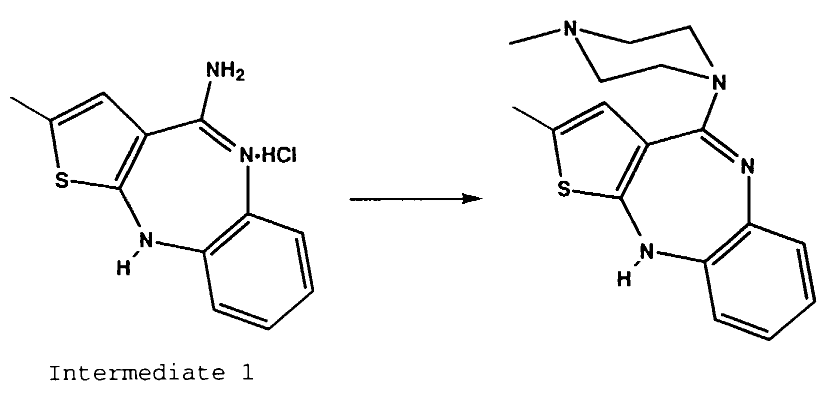

In a suitable three neck flask the following was added:

| | |
|---|---|
| Dimethylsulfoxide (analytical) | 6 volumes |
| Intermediate 1 | 75 g |
| N-Methylpiperazine (reagent) | 6 equivalents |

Intermediate 1 can be prepared using methods known to the skilled artisan. For example, the preparation of the Intermediate 1 is taught in the '382 patent.

A sub-surface nitrogen sparge line was added to remove the ammonia formed during the reaction. The reaction was heated to 120°C and maintained at that temperature throughout the duration of the reaction. The reactions were followed by HPLC until ≤ 5% of the intermediate 1 was left unreacted. After the reaction was complete, the mixture was allowed to cool slowly to 20°C (about 2 hours). Each reaction mixture was then transferred to an appropriate three neck round bottom flask and water bath. To this solution with agitation was added 10 volumes reagent grade methanol and the reaction was stirred at 20°C for 30 minutes. Three volumes of water was added slowly over about 30 minutes. The reaction slurry was cooled to zero to 5°C and stirred for 30 minutes. The product was filtered and the wet cake was washed with chilled methanol. The wet cake was dried in vacuo at 45°C overnight. The product was identified as technical olanzapine. Yield: 76.7%; Potency: 98.1%

### Example 2

### Methanol solvate

A 20 g sample of 2-methyl-4-(4-methyl-1-piperazinyl)-10H-thieno[2,3-b][1,5]benzodiazepine was contacted with a 1:1 (vol) mixture of methanol and water. The mixture was heated to about 78°C and maintained at about 78°C for about 30 minutes. The resulting mixture was allowed to cool to about 30°C. The resulting product was isolated by vacuum filtration and dried for about 30 minutes. The sample was studied using x-ray powder analysis and identified as the methanol solvate of 2-methyl-4-(4-methyl-1-piperazinyl)-10H-thieno[2,3-b] [1,5]benzodiazepine . The thermogravimetric mass loss was 10.1%. Differential Scanning Calorimetry produced an endotherm at 129.7, 133.3, and 195.8 °C.

### Example 3

### Preparation of Technical Grade

The methanol solvate prepared as described *supra.* was dried in a vacuum oven at about 50°C under about 100-300mm vacuum for a period of about 27 hours. The resulting material was identified as technical grade 2-methyl-4-(4-methyl-1-piperazinyl)-10H-thieno[2,3-b][1,5]benzodiazepine using x-ray powder diffraction, DSC, and NMR.

### Example 4

### 1-propanol solvate

A 2.0 g sample of 2-methyl-4-(4-methyl-1-piperazinyl)-10H-thieno[2,3-b][1,5]benzodiazepine was suspended in about 30 g of 1-propanol. The mixture was stirred at about 70°C and maintained at about 70°C for about 30 minutes. The resulting mixture was cooled to about 25°C. The solid product was isolated using vacuum filtration. The wet cake was dried under ambient conditions. Yield: 1.3 g. X-ray powder analysis demonstrated that the product was 1-propanol solvate of 2-methyl-4-(4-methyl-1-piperazinyl)-10H-thieno[2,3-b][1,5]benzodiazepine. Differential Scanning Calorimetry produced endotherms at 84.4 to 96.9, 129.1 to 147.4, and 195.8°C.

### Example 5

### Ethanol solvate

A 2.0 g sample of 2-methyl-4-(4-methyl-1-piperazinyl)-10H-thieno[2,3-b][1,5]benzodiazepine was suspended in absolute ethanol. The stirred suspension was heated to 60°C and maintained at about 60°C for about 30 minutes. The mixture was allowed to cool to about 25°C. The solid product was isolated by vacuum filtration. The wet cake was allowed to dry at about 25°C. The product was identified as the ethanol solvate of 2-methyl-4-(4-methyl-1-piperazinyl)-lOH-thieno[2,3-b][1,5]benzodiazepine using x-ray powder analysis. The thermogravimetric mass loss was 12.7%. Differential Scanning Calorimetry produced an endotherm at 114.8°C and 196.6°C.
Yield: 1.3 g

## Claims

1. A crystalline mono methanol solvate form of a compound of the Formula I

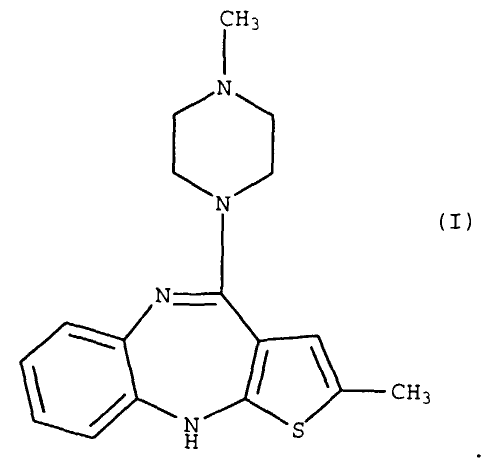

2. A compound as claimed by Claim 1 having a typical X-ray powder diffraction pattern as as represented by the following interplanar spacings:
| **d (A)** |
|---|
| 10.2932 |
| 9.4747 |
| 7.1794 |
| 7.0601 |
| 6.2612 |
| 5.7558 |
| 5.4197 |
| 5.2317 |
| 5.1348 |
| 5.1285 |
| 5.0506 |
| 5.0331 |
| **d** |
|---|
| 4.7672 |
| 4.7137 |
| 4.4533 |
| 4.3315 |
| 4.2656 |
| 4.044 |
| 3.9821 |
| 3.9696 |
| 3.9532 |
| 3.9125 |
| 3.8562 |
| 3.7983 |
| 3.7378 |
| 3.7059 |
| 3.6384 |
| 3.6028 |
| 3.5216 |
| 3.4454 |
| 3.4321 |
| 3.4193 |
| 3.248 |
| 3.2416 |
| 3.1347 |
| 3.127 |
| 3.0121 |
| 2.9979 |
| 2.9767 |
| 2.9303 |
| 2.9172 |
| 2.8048 |
| 2.7483 |
| 2.7412 |

3. A compound as claimed by Claim 2 wherein the compound is substantially pure mono methanol solvate.

4. A crystalline mono ethanol solvate form of a compound of the Formula I

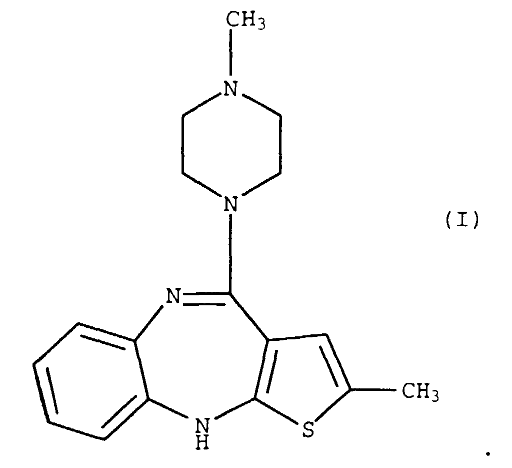

5. A compound as claimed by Claim 4 having a typical X-ray powder diffraction pattern as as represented by the following interplanar spacings:
| **d (A)** |
|---|
| 10.2672 |
| 9.6467 |
| 7.2319 |
| 6.5065 |
| 5.9172 |
| 5.6475 |
| 5.5197 |
| 5.1492 |
| 4.7926 |
| 4.6477 |
| 4.4450 |
| 4.3473 |
| 4.2726 |
| 4.1290 |
| 3.9908 |
| **d** |
|---|
| 3.8338 |
| 3.6932 |
| 3.6189 |
| 3.5070 |
| 3.4389 |
| 3.3083 |
| 3.2618 |
| 3.1950 |
| 3.1050 |
| 3.0761 |
| 3.0477 |
| 2.9835 |
| 2.9292 |
| 2.8703 |
| 2.8215 |
| 2.7852 |
| 2.6908 |
| 2.6287 |
| 2.5791 |

6. A compound as claimed by Claim 5 wherein the compound is substantially pure mono ethanol solvate.

7. A crystalline mono 1-propanol solvate form of a compound of the Formula I

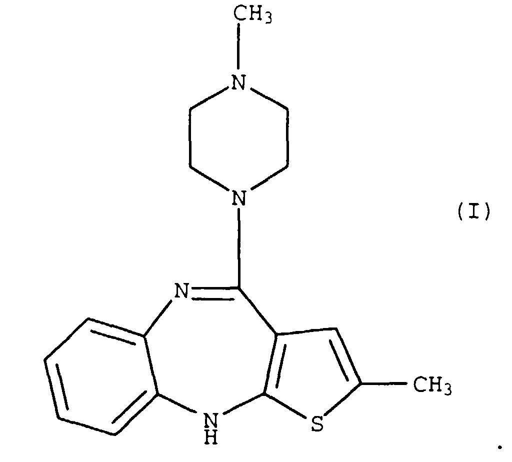

8. A compound as claimed by Claim 7 having a typical X-ray powder diffraction pattern as as represented by the following interplanar spacings:
| **d** |
|---|
| 10.2736 |
| 9.6086 |
| 7.9084 |
| 7.3331 |
| 6.6530 |
| 6.1030 |
| 5.6758 |
| 5.2990 |
| 5.1333 |
| 4.9632 |
| 4.8014 |
| 4.6691 |
| 4.5756 |
| 4.4451 |
| 4.1821 |
| 4.0883 |
| 3.9890 |
| **d** |
|---|
| 3.9021 |
| 3.8234 |
| 3.7258 |
| 3.6785 |
| 3.6033 |
| 3.5265 |
| 3.4234 |
| 3.3653 |
| 3.3121 |
| 3.2125 |
| 3.1795 |
| 3.1230 |
| 3.0435 |
| 2.9461 |
| 2.8951 |
| 2.8495 |
| 2.7744 |
| 2.7445 |
| 2.7140 |
| 2.6609 |
| 2.6110 |
| 2.5683 |

9. A compound as claimed by Claim 8 wherein the compound is substantially pure mono 1-propanol solvate.

10. A process for preparing technical grade olanzapine comprising
crystallizing olanzapine from a reaction mixture using a lower alcohol selected from the group consisting of methanol, ethanol, and 1-propanol to provide the corresponding alcohol solvate of olanzapine; and
drying the resulting solvate.

11. A process as claimed by Claim 10 wherein the lower alcohol is methanol.

## Patentansprüche

1. Kristalline Monomethanolsolvatform einer Verbindung der Formel I

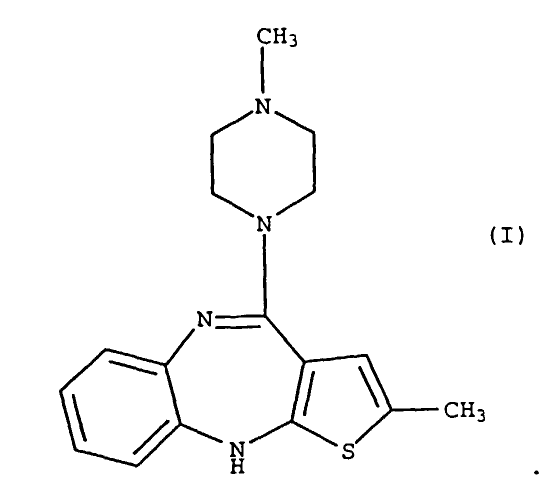

2. Verbindung nach Anspruch 1 mit einem typischen Röntgenbeugungsmuster in Pulverform gemäß der folgenden Interplanarabstände:
| **d in (Å)** |
|---|
| 10,2932 |
| 9,4747 |
| 7,1794 |
| 7,0601 |
| 6,2612 |
| 5,7558 |
| 5,4197 |
| 5,2317 |
| 5,1348 |
| 5,1285 |
| 5,0506 |
| 5,0331 |
| 4,7672 |
| 4,7137 |
| 4,4533 |
| 4,3315 |
| 4,2656 |
| 4,044 |
| 3,9821 |
| 3,9696 |
| 3,9532 |
| 3,9125 |
| 3,8562 |
| 3,7983 |
| 3,7378 |
| 3,7059 |
| 3,6384 |
| 3,6028 |
| 3,5216 |
| 3,4454 |
| 3,4321 |
| 3,4193 |
| 3,248 |
| 3,2416 |
| 3,1347 |
| 3,127 |
| 3,0121 |
| 2,9979 |
| 2,9767 |
| 2,9303 |
| 2,9172 |
| 2,8048 |
| 2,7483 |
| 2,7412 |

3. Verbindung nach Anspruch 2, die im wesentlichen reines Monomethanolsolvat ist.

4. Kristalline Monoethanolsolvatform einer Verbindung der Formel I

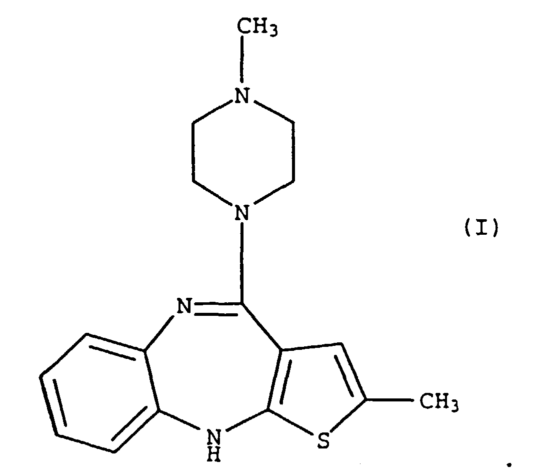

5. Verbindung nach Anspruch 4 mit einem typischen Röntgenbeugungsmuster in Pulverform gemäß der folgenden Interplanarabstände:
| **d in (Å)** |
|---|
| 10,2672 |
| 9,6467 |
| 7,2319 |
| 6,5065 |
| 5,9172 |
| 5,6475 |
| 5,5197 |
| 5,1492 |
| 4,7926 |
| 4,6477 |
| 4,4450 |
| 4,3473 |
| 4,2726 |
| 4,1290 |
| 3,9908 |
| 3,8338 |
| 3,6932 |
| 3,6189 |
| 3,5070 |
| 4,4389 |
| 3,3083 |
| 3,2618 |
| 3,1950 |
| 3,1050 |
| 3,0761 |
| 3,0477 |
| 2,9835 |
| 2,9292 |
| 2,8703 |
| 2,8215 |
| 2,7852 |
| 2,6908 |
| 2,6287 |
| 2,5791 |

6. Verbindung nach Anspruch 5, die im wesentlichen reines Monoethanolsolvat ist.

7. Kristalline Mono-1-propanolsolvatform einer Verbindung der Formel I

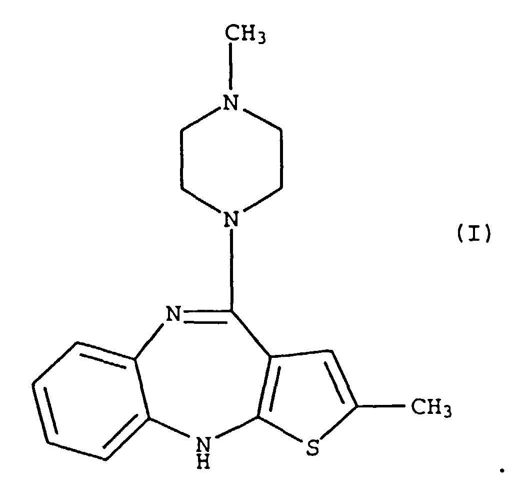

8. Verbindung nach Anspruch 7 mit einem typischen Röntgenbeugungsmuster in Pulverform gemäß der folgenden Interplanarabstände:
| **d in Å** |
|---|
| 10,2736 |
| 9,6086 |
| 7,9084 |
| 7,3331 |
| 6,6530 |
| 6,1030 |
| 5,6758 |
| 5,2990 |
| 5,1333 |
| 4,9632 |
| 4,8014 |
| 4,6691 |
| 4,5756 |
| 4,4451 |
| 4,1821 |
| 4,0883 |
| 3,9890 |
| 3,9021 |
| 3,8234 |
| 3,7258 |
| 3,6785 |
| 3,6033 |
| 3,5265 |
| 3,4234 |
| 3,3653 |
| 3,3121 |
| 3,2125 |
| 3,1795 |
| 3,1230 |
| 3,0435 |
| 2,9461 |
| 2,8951 |
| 2,8495 |
| 2,7744 |
| 2,7445 |
| 2,7140 |
| 2,6609 |
| 2,6110 |
| 2,5683 |

9. Verbindung nach Anspruch 8, die im wesentlichen reines Mono-1-propanolsolvat ist.

10. Verfahren zur Herstellung von technisch reinem Olanzapin, gekennzeichnet durch Kristallisation von Olanzapin aus einem Reaktionsgemisch unter Verwendung eines niederen Alkohols, der ausgewählt ist aus der aus Methanol, Ethanol und 1-Propanol bestehenden Gruppe, unter Bildung des entsprechenden Alkoholsolvats von Olanzapin und Trocknung des erhaltenen Solvats.

11. Verfahren nach Anpruch 10, worin der niedere Alkohol Methanol ist.

## Revendications

1. Forme solvate monométhanol cristalline d'un composé répondant à la Formule I

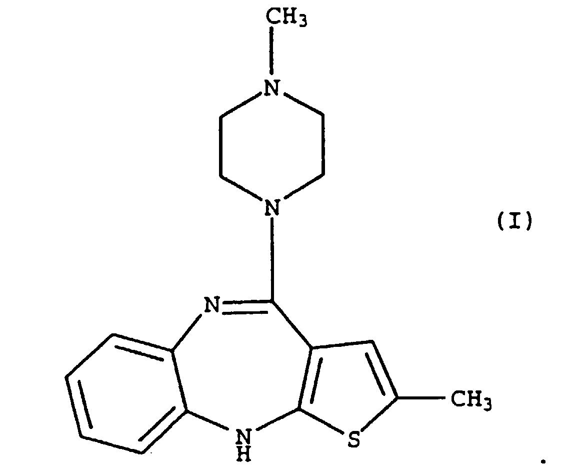

2. Composé selon la revendication 1, présentant un diagramme typique de diffraction des rayons X de la poudre tel que représenté par les espacements interplanaires suivants :
| d(Å) |
|---|
| 10,2932 |
| 9,4747 |
| 7,1794 |
| 7,0601 |
| 6,2612 |
| 5,7558 |
| 5,4197 |
| 5,2317 |
| 5,1348 |
| 5,1285 |
| 5,0506 |
| 5,0331 |
| d |
|---|
| 4,7672 |
| 4,7137 |
| 4,4533 |
| 4,3315 |
| d(Å) |
|---|
| 4,2656 |
| 4,044 |
| 3,9821 |
| 3,9696 |
| 3,9532 |
| 3,9125 |
| 3,8562 |
| 3,7983 |
| 3,7378 |
| 3,7059 |
| 3,6384 |
| 3,6028 |
| 3,5216 |
| 3,4454 |
| 3,4321 |
| 3,4193 |
| 3,248 |
| 3,2416 |
| 3,1347 |
| 3,127 |
| 3,0121 |
| 2,9979 |
| 2,9767 |
| 2,9303 |
| 2,9172 |
| 2,8048 |
| 2,7483 |
| 2,7412. |

3. Composé selon la revendication 2, dans lequel le composé est du solvate monométhanol essentiellement pur.

4. Forme solvate monoéthanol cristalline d'un composé répondant à la Formule I

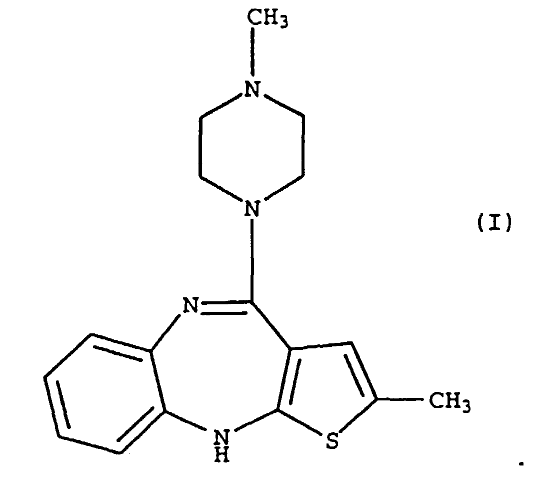

5. Composé selon la revendication 4, présentant un diagramme typique de diffraction des rayons X de la poudre tel que représenté par les espacements interplanaires suivants :
| d(Å) |
|---|
| 10,2672 |
| 9,6467 |
| 7,2319 |
| 6,5065 |
| 5,9172 |
| 5,6475 |
| 5,5197 |
| 5,1492 |
| 4,7926 |
| 4,6477 |
| 4,4450 |
| 4,3473 |
| 4,2726 |
| 4,1290 |
| 3,9908 |
| d |
|---|
| 3,8338 |
| 3,6932 |
| 3,6189 |
| 3,5070 |
| d(Å) |
|---|
| 3,4389 |
| 3,3083 |
| 3,2618 |
| 3,1950 |
| 3,1050 |
| 3,0761 |
| 3,0477 |
| 2,9835 |
| 2,9292 |
| 2,8703 |
| 2,8215 |
| 2,7852 |
| 2,6908 |
| 2,6287 |
| 2,5791. |

6. Composé selon la revendication 5, dans lequel le composé est du solvate monoéthanol essentiellement pur.

7. Forme solvate mono-1-propanol cristalline d'un composé répondant à la Formule I

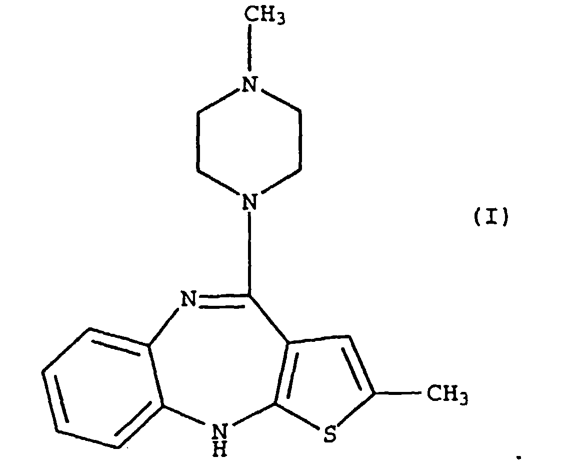

8. Composé selon la revendication 7, présentant un diagramme typique de diffraction des rayons X de la poudre tel que représenté par les espacements interplanaires suivants :
| d |
|---|
| 10,2736 |
| 9,6086 |
| 7,9084 |
| 7,3331 |
| 6,6530 |
| 6,1030 |
| 5,6758 |
| 5,2990 |
| 5,1333 |
| 4,9632 |
| 4,8014 |
| 4,6691 |
| 4,5756 |
| 4,4451 |
| 4,1821 |
| 4,0883 |
| 3,9890 |
| d |
|---|
| 3,9021 |
| 3,8234 |
| 3,7258 |
| 3,6785 |
| 3,6033 |
| 3,5265 |
| 3,4234 |
| 3,3653 |
| 3,3121 |
| 3,2125 |
| 3,1795 |
| 3,1230 |
| 3,0435 |
| 2,9461 |
| 2,8951 |
| 2,8495 |
| 2,7744 |
| 2,7445 |
| 2,7140 |
| 2,6609 |
| 2,6110 |
| 2,5683 |

9. Composé selon la revendication 8, dans lequel le composé est du solvate mono-1-propanol essentiellement pur.

10. Procédé pour préparer de l'olanzapine de qualité technique, comprenant
la cristallisation d'olanzapine à partir d'un mélange réactionnel en utilisant un alcool inférieur choisi dans le groupe constitué du méthanol, de l'éthanol et du 1-propanol de façon à donner le solvate d'alcool d'olanzapine correspondant ; et
le séchage du solvate résultant.

11. Procédé selon la revendication 10, dans lequel l'alcool inférieur est le méthanol.
